# EUROPEAN PATENT SPECIFICATION

(11) **EP 3 165 226 B1**
(45) Date of publication and mention of the grant of the patent: **11.12.2019**
(21) Application number: 15815988.9
(22) Date of filing: 22.06.2015
(51) Int. Cl.: A61K 35/30, A61K 35/36, A61K 38/17, A61P 35/00, A61P 17/00

(54) **PROTEIN-POLYPEPTIDE COMPLEX FOR USE IN TREATING PATIENTS HAVING CANCEROUS DISEASES.**
PROTEIN-POLYPEPTIDE COMPLEX ZUR VERWENDUNG BEI DER BEHANDLUNG VON KREBSARTIGEN HAUTERKRANKUNGEN.
COMPLEXE DE PROTÉINE-POLYPEPTIDE DESTINÉES À ÊTRE UTILISÉES DANS LE TRAITEMENT DE PATIENTS SOUFFRANT D'AFFECTIONS ONCOLOGIQUES DE LA PEAU

(30) Priority: 01.07.2014 RU 2014126510
(43) Date of publication of application: 10.05.2017
(73) Proprietor: Vasilana Holdings Limited, 1070 Nicosia (CY); Nazarenko, Anna Borisovna, Moscow 121614 (RU)
(72) Inventor: NAZARENKO, Anna Borisovna, Moscow 121614 (RU); SOKOLOV, Mikhail Anatolevich, Moscow 107150 (RU)
(74) Representative: Held, Stephan
(86) International application number: PCT/RU2015/000382
(87) International publication number: WO 2016/003320

(56) References cited:
- WO-A1-2009/043459
- RU-C1- 2 283 129
- RU-C1- 2 451 531
- RU-C1- 2 460 736
- RU-C2- 2 446 792
- RU-C2- 2 477 637
- RU-C2- 2 485 133

## Description

### The art

The invention refers to medicine, in particular to oncology, pharmacology and veterinary science, and can be used in the treatment and prevention of neoplastic processes of skin tissues - squamous-cell and basal-cell skin cancer, as well as melanoma and other benignant and malignant skin tumors, with the help of pharmaceutical preparation including protein-polypeptide complex produced from homogenates of nervous and skin tissues of the hoofed livestock.

At present time, on the background of steady growth of skin tissue diseases of autoimmune, vascular, toxic, infectious, traumatic origin, and iatrogenic complications after use of "rejuvenating" preparations, cosmetic products and procedures, there are no preparations in the group of direct reparative agents specific for the skin tissue.

### Prior art

Different forms of oncological skin diseases (skin cancer) are known, such as melanoma, basalioma (basal-cell cancer), squamous-cell skin cancer.

Treatment of melanomas, in particular, represents a challenge due to its rapid and early dissemination. It shall be performed only in the conditions of a special treatment institution. Melanoma treatment is primarily surgical. At the 1^{st} and 2^{nd} stages extensive melanoma excision is performed.

Some vaccines against melanoma are known, which are represented as allogeneic tumor cells and their lysates, autogenic tumor cells, different antigens (MAGE-1, MAGE-3, MART-1, gr100. etc.). These vaccines increase activity of cytotoxic lymphocytes against malignized cells and activate proliferation of T-lymphocytes and infiltration of CD8+ tumor.

It is known that anti-cancer drugs are divided into several groups according to their mechanism of action and chemical structure: alkylating compounds, antimetabolites, antibiotics, substances of plant or animal origin, hormonal drugs, ferments, etc.

The anti-tumor action of such compounds is based on violation (suppression) of exchange of nucleic acids, especially nucleotides, which results in termination of cellular pressure and tumor growth (patent US 4622325, 11.11.1986; author's certificate SU 1208619, patent RU 2040255).

However, due to the fact that anti-tumor drugs contribute only to suppression of cellular activity and do not touch causes inducing this tumor, recurrence of tumor and metastasis cannot be excluded.

Basalioma is a skin cancer with locally destructing growth, which arises from epidermis or hair follicles and rarely leads to metastasis (according to WHO in 1974). According to a number of authors (Shanin A.P. Skin tumors, their origin, clinical picture and treatment, L., 1969, p.154-172), basalioma occurs more frequently than other skin neoplasms and occupies intermediate position between benignant tumors and squamous-cell cancer. It accounts from 77 to 90% of malignant epithelial skin tumors (Venkei T., Shuvgar Y. Malignant skin tumors, Budapest, 1962, p. 12-15). Known methods of skin basalioma treatment are: surgical excision, electrocoagulation, cryodestruction, radiation therapy, laser therapy, chemotherapy, external treatment by anti-tumor drugs (Kogan M.G. Improvement of methods of treating multiple skin basaliomas on the basis of their immunomorphological peculiarities study. Thesis abstract of the candidate of medical sciences, M., 1984, p. 24) are not effective enough, induce recurrences of disease and accompanied by delayed post-surgery complications in the form of deep skin defects.

Method of complex treatment of skin basaliomas in known from RU 2179000 dd. 10.02.2002.

It is suggested to start treatment from taking human leukocyte interferon in the form of inhalations in the dose of 20000 IU B every other day, treatment course consists of 20 procedures. On the background of immune correction, radiowave remobal of basaliomas was performed by Surgitron instrument by Ellman international (USA). This method provides a qualitatively new approach and high efficiency in complex therapy of skin basaliomas and correction of immunological indicators, leads to a high rate of healing and is characterized by the absence of recurrences and therapy complications.

However, this method does not have a broad spectrum upon development of neoplastic and reparative skin processes and upon treatment and preventive treatment of various oncological skin diseases.

A special area in the therapy of malignant tumors is the development of cancer-treating methods with the use of drugs produced from raw animal materials, mainly from embryonic organs of animals and humans (DE 228739, 29.04.1983; DE 2600442, 08.01.1976).

Drugs produced from animal tissues possess a significant advantage against known cytostatic drugs of chemical origin, since they possess weak hepatotoxic and teratogenic effect. However, use of such drugs is low due to limited raw materials base and the risk of allergic complications, since the latter possess visible antigenic specificity.

In particular, a method of cancer treatment with the help of embryonic tissue homogenate is known from the application of France No. 2451193 dd. 13.03.1979.

Known method consists of the following: upon external localization of neoplastic process, homogenate (suspension) of embryonic tissue mixed with pharmaceutically suitable filler is attached to the patient in the form of applications, in case of internal tumor localization, 10 ml of 10%-salt-and-water homogenate of embryonic tissue is administered parenterally 1-2 times per day during 4-12 weeks. Isotonic sodium chloride solution is used as a filler. After month of treatment, frequency of embryonic tissue homogenate administration is gradually reduced to 1-2 times per week, and treatment is continued 2-3 months more. This method is especially effective upon external tumor localization, since tumor separation from adjacent healthy tissues is frequently visible, which contributes to successful surgical removal of the tumor. The closes anti-tumor effect is visible 2-3 weeks after treatment commencement. Average remission duration is 9 months.

A disadvantage of the known method is the fact that the use of xenogenic embryonic tissue is limited by the special antigenic specificity. Besides, the suggested embryonic tissue homogenate is a mix of undetermined composition containing fetal proteins, peptides, nucleic acids and other components. Therefore, the suggested agent cannot be standardized in composition, since it will depend on the initial tissue, and adverse complications can arise during treatment process due to toxic reactions.

RU 2105567 dd. 27.02.1998 describes method of treating malignant tumors, including cancer of skin, lip, oral mucosa, upon persistent wounds of trophic ulcers by administration of alpha-fetoprotein (AFP) in a single dose of 500-5000 IU/kg of patient's weight 1-2 times daily during 4-6 weeks.

Protein quantity equal to 1.25 ng is accepted as IU (international unit) of AFP. Hereinafter all data will be provided in IUs.

AFP is an oncofetal protein produced from abortive human material (natural alpha-fetoprotein) or from vaccinia virus on the embryonic cells of human lungs (recombinant alpha-fetoprotein). The claimed method can use any type of alpha-fetoprotein (either natural or recombinant). AFP is not pyrogenic and does not cumulate in the organism, does not possess teratogenic, anaphylactogenic, embryotoxic, allergizing and histamine-like properties.

However, this method is based on the use of abortive human material, which limits its use.

RU 2240810 dd. 27.11.2004 describes method of prevention and treatment of pre-tumor pathological conditions, including vaccination of persons belonging to the groups of high oncological risk according to testing results, by embryonic anti-tumor modulator (EATM) subcutaneously once per year in the dose of 0.02 - 0.04 mg/kg. EATM contains a wide spectrum of oncofetal antigens. The drug is produced from embryonic tissues of humans and animals and contains a pool of proteins-glyco-proteins derived by alcohol precipitation method. However, this drug is not used for treatment and prevention (preventive treatment) of skin cancer.

RU2283129 dd. 10.09.2006 describes protein-peptide anti-tumor preparation (composition) containing heat shock protein of HSP70 family and non-covalent tumor-specific peptide selected from the group: MAGE-A1, - A2, - A3, etc.. 100, HER2/neu, EGFRvIII or fragment of any of them.

This cellular preparation is used in the method of immune therapy and tumors immunoprophylaxis.

Therefore, as follows from the prior art, various protein-peptide drugs are widely used in medicine and pharmacology.

Thus, in particular, RU 2485132 dd. 20.06.2013 describes protein-polypeptide complex (PPC) possessing antihypoxic, tissue-specific reparative action on the central and peripheral nervous system, and the pharmaceutical composition based thereon.

PPC is produced from nervous and skin tissues of hoofed livestock embryos.

It is not used for skin cancer treatment.

### Invention disclosure

The technical problem of the claimed invention is the increase of effectiveness of treatment and prevention of neoplastic processes in skin tissues, as well as the extension of the range of means for treating oncological diseases with the use of such embryonic protein-polypeptide complex possessing the properties of regulator of cellular differentiation and high general biological activity.

Technical result is achieved by the claimed inventions groups including methods of treating patients with oncological skin diseases (melanoma, basalioma, squamous-cell cancer) (options), as well as the method of preventive treatment of oncological skin diseases upon development of neoplastic processes of skin tissue, such as melanoma, basalioma, squamous-cell cancer.

In the claimed invention, the known protein-polypeptide complex is used as a primary active substance (biologically active substance), it is represented as a biologically active protein-polypeptide complex (hereinafter - PPC) with the molecular mass of its constituent components within the range of 5-200 kDa and content of medium molecular fraction within the range of 10 to 120 kDa in at least 80%, general protein content 0.2 - 4.2 mg/ml, maximum absorption peak of UV spectrum of solution at the wave length 280±5nm, peak presence at the wave length 274-284 nm in the UV-visible spectrum, presence of specific bands within pl range from 4.2 to 8.4 at isoelectric focusing in a 5% polyacrylamide gel and pharmaceutically acceptable agent, for example diluents, as well as conventional agents (pharmaceutically acceptable) upon composition of pastes, gels, creams, etc., and the diluents can include buffer solution and excipients - high-molecular compounds belonging to the class of preservatives and solubilizers in sufficient concentrations. The substance is produced by ion-exchange chromatography of filtrate of extracted homogenate of skin, cerebral and spinal cord tissues of hoofed livestock's embryos with the gestation period from the middle of the first third till the middle of the last third in the presence of buffer solution, detergents, proteolysis inhibitors and solubilizers.

Biologically active protein-polypeptide complex used in the claimed inventions group and method of its production are protected by the applicant of this invention and described in RU 2428196 dd.10.09.2011.

Along with that, in RU 2445106, 20.03.2012 the applicant of this invention protects pharmaceutical composition intended for treating diseases of the central and peripheral nervous system of vascular, traumatic, hypoxic and autoimmune origin, which contains specified biologically active protein-polypeptide complex as an active substance.

As the result of supplementary research, applicants of the claimed inventions (inventions group) identified possibility of its use as an active substance in the method of prevention and treatment upon development of neoplastic and reparative skin processes, oncological skin diseases, such as melanoma, basalioma (basal-cell cancer) and squamous-cell cancer.

As the result of additional researches and experiments, the applicant developed regimen for treating such diseases with the use of the specified protein-polypeptide complex possessing certain specified characteristics.

Attempts to use extracts of embryonic tissues for treating and prevention of oncological diseases in general and on skin in particular are known from time immemorial. They are all combined by the expresses anti-tumor action of the proteins - differentiation factors, produced by embryonic skin cells. Primary function of such proteins upon rapid and simultaneous division of a huge number of cells for active growth of the organism in general is the provision of correct cells differentiation and their timely apoptosis.

The main feature characterizing tumor cells is violation of final differentiation and loss of apoptosis ability.

The main biological molecules ensuring primary signaling to evolutionally fixed ways of development and differentiation of tissues in mammals' embryogenesis are proteins and polypeptides. Production of fractions of native protein and polypeptide ensembles from embryonic tissues is quite a difficult and expensive problem, the solution of which depends on multiple sequential actions requiring accurate fulfillment of each regulation paragraph in strictly defined conditions, since expressiveness of target fractions anti-tumor action, their toxicity (allergenicity, immunotozicity) and stability directly depend on the nativeness degree of the produced protein-polypeptide molecules.

The most appropriate solution for preservation of nativeness and stability of differentiation factors and their application in the therapy of neoplastic skin diseases turned out to be the method of protein-polypeptide complex production, which allows to obtain protein-polypeptide complex possessing necessary properties allowing to use it in the claimed prevention and treatment method upon development of neoplastic and reparative skin processes, oncological ski disease (method of producing this protein-polypeptide complex and the protein-polypeptide complex itself are described in the patent RU 2428196, as was noted above).

Thus, technical result of the claimed inventions group is the increase of effectiveness upon treatment of oncological skin disease and their prevention through the use of biologically active PPC possessing tissue-specific reparative actions on skin tissue and having expressed healing and preventive effect upon therapy of neoplastic diseases of the skin tissue.

The defined technical problem and the achieved technical result are ensured by the claimed inventions group.

The claimed group of inventions includes method of treating patients with oncological skin diseases, including determination of tumor sizes and, depending on its size, administration of intracutaneous injections around the ski tissue lesion prior to and/or after tumor removal, leaving 2-3 mm from its edge, or directly into tumor tissue by even administration with the needle's reverse run 1-3 times per week in the form of 6-20 injections during the course of 4-14 procedures with medication in the form of 1.0-2.5 ml solution of protein-polypeptide complex in pharmaceutically acceptable carrier with complex content (concentration) in it in the amount of 0.05 - 0.4 mg; along with that, protein-polypeptide complex is used as an active substance, it is produced from homogenate of embryonic nervous and skin tissues of the hoofed livestock, and containing negatively charged slightly acidic and neutral proteins and polypeptides with the molecular mass from 5 to 200 kDa and fraction content with the average molecular mass from 10 to 120 kDa of at least 80%, general protein content 0.2 - 4.2 mg/ml, maximum absorption peak of UV spectrum of solution at the wave length 280±5nm, peak presence at the wave length 274-284 nm in the UV-visible spectrum, presence of specific bands within pl range from 4.2 to 8.4 at isoelectric focusing in a 5% polyacrylamide gel.

This, this method of treating patients with oncological skin diseases (melanoma, basal-cell and squamous-cell cancer) consists in the application of active substance in the form of protein-polypeptide complex produced from homogenate of embryonic nervous and skin tissues of the hoofed livestock and consisting of negatively charged slightly acidic and neutral proteins and polypeptides with the molecular mass from 5 to 200 kDa. After determination of the tumor size (for example, by ultrasound or other known methods), intracutaneous injections are made with protein-polypeptide complex solution around the lesion and directly into tumor tissue prior to its surgical removal (by the method of classic or radio-wave surgery, photodynamic therapy or other known methods), as well as after removal - around skin wound, leaving 2-3 mm space from its edge.

Diluent containing buffer solution and excipients, such as aspartate buffer, phosphate buffer, glycine buffer, etc., is used as a pharmaceutically acceptable carrier.

Along with that, the following recommendations are prescribed:
- For tumor size up to 1 cm, during one procedure prior to lesion removal - no more than 0.1 mg of protein-polypeptide complex in 1.0 ml of pharmaceutically acceptable diluent is administered in the form of 6-8 injections 1-2 times per week, the course is 4-6 procedures;
- For tumor size from 1 cm to 1.5 cm, during one procedure no more than 0.15 mg of protein-polypeptide complex in 1.0-1.5 ml of pharmaceutically acceptable diluent is administered in the form of 10-14 injections 2-3 times per week, the course is 6-9 procedures;
- For tumor size from 1.5 cm to 2.0 cm, during one procedure no more than 0.25 mg of protein-polypeptide complex in 1.5-2.0 ml of pharmaceutically acceptable diluent is administered in the form of 12-16 injections 2-3 times per week, the course is 9-12 procedures;
- For tumor size from 2.0 cm and higher, during one procedure up to 0.4 mg of protein-polypeptide complex in 2.5-4.0 ml of pharmaceutically acceptable diluent is administered in the form of 16-20 injections at least 3 times per week, the course is at least 12 procedures;

Protein-polypeptide complex can be administered together with other immunomodulators, angiogenesis suppressors, substances suppressing tumor cells growth, such as DNA and RNA polynucleotides; chaperones HSP 60, HSP 70, HSP 90, tumor-specific peptides from the MAGE group, such as MAGE-A2, MAGE-A3 and gp 100, HER2/neu, EGFRvlll or their ferments; cytostatics and other inhibitors of tumor growth in the effective doses.

Apart from the local administration, systemic administration of protein-polypeptide complex is also made subcutaneously once daily or once in 2-3 days, courses - 10-14 days, 0.1-0.4 mg daily, with the 10-28-days interval between courses, depending on the expressiveness of neoplastic process, its localization, intensity of primary treatment and the local therapy course.

Another invention from the claimed group is the method for use in treating patients with oncological skin diseases, including determination of the tumor size and, depending on the size, performance of external applications by pharmaceutical preparation, including protein-polypeptide complex and pharmaceutically acceptable carrier, directly on the tumor tissue and around its lesion prior to surgical removal, as well as after tumor removal, directly on the skin wound and around it at 3.5 mm from the edge, and the applications are made 3-4 times per week within the course of 12-16 procedures; the specified protein-polypeptide complex is used in the concentration 0.05 - 0.4 mg with pharmaceutically acceptable carrier in the amount of 0.5 - 2.5 ml.

This method is also intended for use in treating patients with oncological skin diseases (melanoma, basal-cell and squamous-cell cancer). After determination of tumor sizes (for example, by ultrasound or other known methods), external applications are made by protein-polypeptide complex solution directly on the tumor tissue and around its lesion prior to surgical removal (by the method of classic or radio-wave surgery, photodynamic therapy or other known methods), as well as after removal, directly on the skin wound at 3-5 mm from its edge.

If using treatment method with applications, this method is preferably to perform as follows:
- For tumor size up to 1 cm, during one procedure 0.05-0.1 mg of protein-polypeptide complex is used in 1.0 ml of pharmaceutically acceptable diluent (carrier), 3-4 applications per week within the course of 12-16 procedures;
- For tumor size from 1 cm to 1.5 cm, during one procedure no more than 0.15 mg of protein-polypeptide complex is used in 1.0-1.5 ml of pharmaceutically acceptable diluent (carrier), 3-4 applications per week within the course of 12-16 procedures;
- For tumor size from 1.5 to 2.0 cm, during one procedure no more than 0.25-3.0 mg of protein-polypeptide complex is used in 1.5-2.0 ml of pharmaceutically acceptable diluent (carrier), 3-4 applications per week within the course of 12-16 procedures;
- For tumor size from 2.0 cm, during one procedure 0.4 mg of protein-polypeptide complex is used in 2.0-3.0 ml of pharmaceutically acceptable diluent (carrier), 4 applications per week within the course of 12-16 procedures;

Protein-polypeptide complex in that case (in the "method" option) is used in the form of paste or gel with preservation of the specified concentrations, volumetric proportions, single and course dosage regimens.

Hydrophilic pharmaceutically acceptable carrier (for example, Vaseline, lanoline, polyethyleneglycol, etc.) is used as a paste or gel base.

When treating by applications, protein-polypeptide complex can be used in the form of liposomal emulsion individually or within composition of a paste or gel with preservation of the same concentration, volumetric proportions, single and course dosage regimens.

Upon implementation of the method protein-polypeptide complex administration through the skin, the method of electrophoresis or phonophoresis is used individually or within composition of liposomal emulsion, paste or gel with preservation of the same concentration, volumetric proportions, single and course dosage regimens.

Upon making applications, protein-polypeptide complex is administered together with other immunomodulators, angiongenesis suppressors, substances suppressing tumor cells growth, such as DNA and RNA polynucleotides; chaperones HSP 60, HSP 70, HSP 90, tumor-specific peptides from the MAGE group, such as MAGE-A2, MAGE-A3, gp 100, HER2/neu, EGFRvlll or their ferments; cytostatics (and other inhibitors of tumor growth) in the effective doses.

Another invention from the claimed group is the method of preventive treatment upon development of neoplastic processes of skin tissue, such as melanoma, basal-cell and squamous-cell cancer, including use of pharmaceutical preparation including protein-polypeptide complex and pharmaceutically acceptable carriers in the form of cosmetic compositions, such as day and night creams, gels, lotions individually or in the liposomal form, as well as in the form of photoactive or photoprotective compositions, daily, in the concentration 0.001-0.05 mg/ml, 1-2 times daily with the single dose, 10-20 days, at least 4 courses per year.

Antioneoplastic activity upon direct use of PPC within pharmaceutical compositions, BAA (biologically active additives) is expresses in the activation of apoptosis and necrosis cascades of tumor cells with simultaneous stimulation of autophagy processes. Complex action mechanisms were proved in cell experiments in vitro on cell lines, in tests with finite tumor cell lines on laboratory animals, in the clinic among patients with basal-cell, squamous-cell cancer and melanoma.

### The best option of invention implementation

Examples illustrating the claimed inventions group but not limiting it are provided below.

In this regard, for example, concentration (content) of protein-polypeptide complex (PPC) not more than 0.1 mg/ml (Example 2) indicates to the use of 0.5-2.5 ml of composition containing 0.05 - 0.25 mg of PPC.

Pharmaceutical preparation (used) is usually released as a solution 0.1 mg/ml, on the basis of which pharmaceutical preparations are produced in the form of solutions, pastes, gels, creams, lotions, liposomal emulsions with necessary PPC concentrations in them in accordance with the claimed methods in pharmaceutically acceptable carriers in combination, if required, with auxiliary additives (target additives) traditionally used in medicine, in pharmaceutical industry and, for example (in particular, according to example 2), 0.1 mg/ml means 10 mg of PPC in 100 ml, i.e. 0,01% PPC solution (with account of standard deviation for water density (as a pharmaceutically acceptable carrier) or paste, gel base, etc.

### Example 1.

Female patient R., 49 years, feels sick for about 4 months. Addressed to polyclinic.

### Statuslocalis

Eye corner skin has a pink-colored plaque, d = 1.5 cm, with haemorrhagic crusting in the center.

### Preliminary diagnosis

Left eye corner basalioma of II degree, II clinical group
Scraping of left eye corner plaque was made. Cytology No. 1217/12,
Finding: basalioma
Treatment: applications of protein-polypeptide complex are made in 50 mM glycine-phosphate buffer with protein concentration 0.1 mg/ml, and in amount of 1.5 ml directly on the damaged tissue and around it, at 5 mm from the edge, daily within the course of 14 procedures, with repeated course in 10-14 days. The complex was applied on a sterile four-layer gauze pad, skin in the lesion area was preliminary cleaned by 20% alcohol solution.
After applications course the patient was referred for treatment to the radio-ray therapy department. Upon radiation therapy completion, the same 2 courses of applications were repeated with 14-days interval between the courses.
3-year follow-up by oncodermatologist confirmed complete remission of disease.

### Example 2.

Male patient, 34 years old, during 3 years observes occurrence and enlargement of a neoplasm in the form of a pink stain up to 1.0 cm in diameter with red induration in the center.

### Statuslocalis

On the forehead skin, in the center, closely to the hairy part, a flat and slightly sunken erosion of red color is identified, it is moist in the center and has up to 1.2 cm diameter.

### Preliminary diagnosis

Forehead skin basalioma of II degree
Erosion scrape was made. Cytology No. 292/13, finding: basalioma.
Treatment: paste composition consisting of polyethylene glycol 400 and 1500 in the ratio 3:1 and protein-polypeptide complex in the concentration 0.1 mg/ml, was applied directly to the affected skin area and around it, at 5 mm from the edges, once daily during 12 days with repeated course in 7 - 10 days, up to 6 double courses per year. The composition was applied as a thin layer on a sterile gauze pad fixed by plaster.

After annual course, tumor diameter reduced to 3 mm, and the patient was referred for treatment to photodynamic therapy department, where 1 procedure with Radahlorin was performed. In 3 weeks, after bandage removal, hardly visible pink stain is noted in the lesion area, which is weekly distinguished from the adjacent skin sections. 2-year follow-up by oncodermatologist confirmed complete remission of the disease.

### Example 3.

Male patient, 55 years old. Feels sick for more than 4-5 years. Practiced self-treatment. Complaints on admission: presence of ulcerous defect in the right popliteal area.

### Statuslocalis

Skin on the right popliteal area has an ulcerous defect with spread along rear surface of the internal and the external part of the hip and the leg. Ulcer with degradation signs and heavy purulent discharges.

Histopathological report: squamous-cell cancer.

Instrumental examination: according to results of ultrasound examination, X-ray examination and inspection - distant metastases are not identified.

Clinical diagnosis: squamous-cell cancer of the left popliteal area skin, exophytic form, T2N0M0.

Treatment: tumor removal with further single infiltration soaking of health tissues by 0.1% PPC solution - 5.0 ml in 50 mM glycine-phosphate buffer at 0.5 - 1.0 cm from the lesion. Bandages with sterile liposomal emulsion containing 0.1% PPC in 20 mM aspartate buffer solution.

Surgical wound is healed by immediate union. The patient was referred to radiation treatment in the radiological department, and upon its completion, the patients was discharged for follow-up by the oncologist. In the outpatient mode systematic administration of protein-polypeptide complex subcutaneously was continued by 14-day courses, 0.1 mg daily, with 28-day break between courses during 1 year. 3-year follow-up by oncodermatologist confirmed complete remission of the disease.

### Example 4.

Determination of PPC effect of the growth of the human skin normal fibroblasts (FBH) and B16 melanoma cells.

### Method.

*Cells culture.* B16 melanoma cells were obtained from the collection of the Institute of Molecular Biology of RAS. Skin fibroblasts of healthy people (FBH) used in the experiments only on 2-8 passages, were obtained from the biotechnology laboratory of the I.M. Secehenov's 1^{st} MSMU. Cells cultivation was performed in standard conditions (5% CO₂, 37°C). DMEM medium and fetal calf serum (FCS) were used (by «Gibco», USA, received via Invitrogen company, Moscow).

To determine cytotoxic anti-tumor activity of PPC, the cells were subcultured in 96-well plates (Costar) in 100 µl of medium with density 5 x 10³ cells/well. The cells were pre-incubated in plates during 24 hours for adaptation before preparations addition. PPC was added to cells within the concentration range from 0.001 to 0.2 mg/ml for 4 times, and in 72 hours the experiments was terminated. The number of surviving metabolically active cells was measured with the use of MTT-test. The method is based on the fact that mitochondria's dehydrogenases only of metabolically active cells convert MTT-reagent into colored formazan crystals. After that, 10 µl of 0.5% MTT solution was added to cultured cells, and then they were incubated during 3 hours at 37° C. Then formazan crystals were diluted in 0.1 ml of DMSO with shaking by Titramax 101. Optical density of solutions was measured by multiscan EX («Lab. System», Finland) at wave length 540 nm. The number of surviving cells was calculated in percentage of control, which was represented by cells cultivated without addition of L-asparaginases preparations.

Flow-cytofluorometric analysis of the cells cycle and determination of the number of apoptotic cells after treatment of FBH and tumor cells by PPC were performed according to the standard method (Griffin C., Hamm C., McNulty J., Pandey S. Pancratistatin induces apoptosis in clinical leukemia samples with minimal effect on non-cancerous peripheral blood mononuclear cells // Cancer. Cell. Int. - 2010. - Vol. 10. - PP.6. doi:10.1186/1475-2867-10-6).

For that purpose, fibroblasts and tumor cells (0,5 x 10⁶) were sub-cultured into mats of 25-cm² volume, containing 5 ml of the growth medium and then were cultivated in the standard conditions. 24 hours after, PPC preparations were added to the medium. 72 hours after, cells sediment was collected for analysis in cytofluorometer after their treatment by iodic propidium (Sigma). For flow cytofluorometric analysis performance, cytofluorometer FACSAria I (Becton Dickinson, USA) was used.

Statistical processing of results was performed with the use of Statistica 6.0 program. Comparison was performed with the use of non-parametric Mann-Whitney method.

### Results

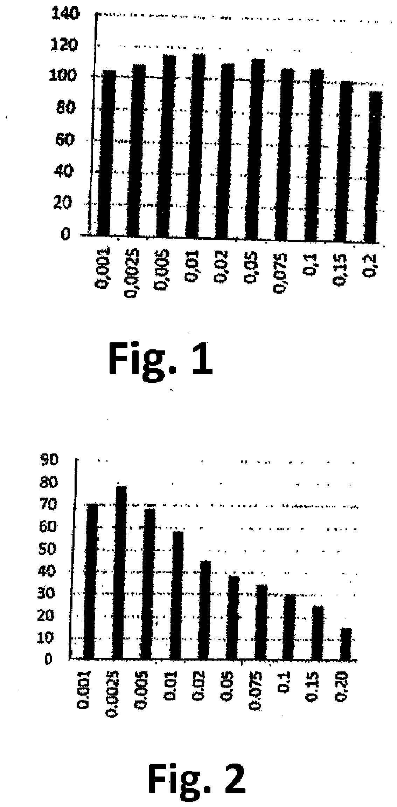

Results are illustrated on the figures 1 and 2, where percent (%) of surviving cells is shown along the X-axis, and PPC concentration (mg) in the cells cultivation medium is shown along the Y-axis.
Fig. 1 shows data about cytofluorometric research of human fibroblast cells (FBH) 72 hours after addition of preparation under his invention into growth medium (trade name - Cellex).
Fig. 2 shows data about cytofluorometric research of B16 melanoma cells 72 hours after addition of preparation under his invention into growth medium (trade name - Cellex).

Results presented on the fig. 1 evidence that PPC added to human skin fibroblasts does not produce any inhibiting effect on the growth of these cells even in 72 hours.

At the same time, in 72 hours PPC suppresses growth of B16 melanoma cells (fig. 2). Cytofluorometric research showed absence of apoptotic cells in B16 melanoma samples in 72 hours of experiment, most likely the cells died in the result of necrosis or authophagy.

### Example 5.

Male patient, 63 years old. Feels sick for several months. Exact disease onset: January 30, 2012. During several recent months noted enlargement of pigmental neoplasm, appearance of exulcerations, erosions (initial attendance).
*Complaints on admission:* presence of neoplasm on the chest skin, bleeding from neoplasm

### Statuslocalis

Primary rash elements: lump.

### Rash location: chest

*Clinical diagnosis:* chest skin melanoma T2N1M0. Immediately after surgery (tumor removal), systematic administration of protein-polypeptide complex was performed by subcutaneous injections during 14 days, in 0.2 mg solution, once daily, with 14-day break between the courses, 6 courses in total. Surgical wound was healed by immediate union, stitches were taken out on the 6^{th} day, the stitch is soft and hardly visible.

2-year follow-up by oncodermatologist confirmed complete remission of the disease.

### Example 6.

Female patient L., 66 years old.
Feels sick for 7 years. Three years ago, she treated lesion of "keratoma" (as she thought) by "super-celandine", and after inflammatory exulceration the node split into two nodes.

### Statuslocalis

Localized rash. On the external part of the right hip the node of irregular form is about 1.5 cm in diameter.
*Clinical diagnosis:* Nodal melanoma of the right hip.

### Histopathological report:

Nodal melanoma from epithelium-like cells with epidermis exulceration, expressed lymphoid infiltration, with invasion of Clark's II-III level and depth up to 2.2 according to Breslow. The tumor was removed within the limits of the healthy tissues.

Immediately after surgery (tumor removal), systematic administration of protein-polypeptide complex was performed by injections within 14-day courses, 0.1 mg once daily, with 14-day break between the course, in total - 4 courses, and external application of 0.1% PPC to post-surgery wound area within the composition of the sterile hydrophilic paste from polyethyleneglycoles 400 and 1200 in the ratio 3:1, accordingly, within 7 days. Surgical wound was healed by immediate union, stitches were removed on the 7^{th} day.

3-year follow-up by oncodermatologist confirmed complete remission of the disease.

### Industrial applicability

Therefore, as follows from the provided examples, claimed group of inventions ensures implementation of the method of cancer prevention and treatment with the use of protein-polypeptide preparation with certain characteristics, in certain doses and with certain treatment regimen, ensuring efficacy of these methods, which vividly demonstrates the applicability of the claimed inventions group.

## Claims

1. Protein-polypeptide complex for use in treating oncological skin diseases as a biologically active ingredient within the composition of the medication in the form of 1.0-2.5 ml solution in a pharmaceutically acceptable carrier with complex concentration in it in the amount of 0.05 - 0.4 mg and injected with the needle's reverse run 1-3 times per week in the form of 6-20 injections during the course of 4-14 procedures during intracutaneous injections around lesion of the damaged tissue prior and/or after tumor removal, or directly into the tumor tissue after tumor size determination; along with that, the protein-polypeptide complex is used as an active substance, it is produced from homogenate of embryonic nervous and skin tissues of the hoofed livestock and contains negatively charged slightly acidic and neutral proteins and polypeptides with the molecular mass from 5 to 200 kDa and fraction content with the average molecular mass within the range from 10 to 120 kDa of at least 80 %, general protein content 0.2 - 4.2 mg/ml, maximum absorption peak of the solution UV spectrum at the wavelength 280±5nm, peak presence at the wave length 274-284 nm in the UV-visible spectrum range, presence of specific bands within pl range from 4.2 to 8.4 at isoelectric focusing in 5% polyacrylamide gel.

2. Protein-polypeptide complex for use in treating oncological skin diseases as a biologically active ingredient within the composition of the medication according to claim 1, **characterized in that** the diluent containing buffer solution and excipients is used as a pharmaceutically acceptable carrier.

3. Protein-polypeptide complex for use in treating oncological skin diseases as a biologically active ingredient within the composition of the medication according to claim 1, **characterized in that**:
- for tumor size up to 1 cm, one procedure prior to lesion removal no more than 0.1 mg of the protein-polypeptide complex in 1.0 ml of the pharmaceutically acceptable diluent is administered in the form of 6-8 injections 1-2 times per week, the course is 4-6 procedures;
- for tumor size up from 1 to 1.5 cm, during 1 procedure no more than 0.15 mg of the protein-polypeptide complex in 1.0-1.5 ml of the pharmaceutically acceptable diluent is administered in the form of 10-14 injections 2-3 times per week;
- For tumor size from 1.5 cm to 2.0 cm, during one procedure no more than 0.25 mg of the protein-polypeptide complex in 1.5-2.0 ml of the pharmaceutically acceptable diluent is administered in the form of 12-16 injections 2-3 times per week, the course is 9-12 procedures;
- For tumor size from 2.0 cm and higher, during one procedure up to 0.4 mg of the protein-polypeptide complex in 2.5-4.0 ml of the pharmaceutically acceptable diluent is administered in the form of 16-20 injections at least 3 times per week, the course is 12 procedures.

4. Protein-polypeptide complex used for treating oncological skin diseases as a biologically active ingredient within the composition of the medication under the claim 1, **characterized in that** it is administered in combination with other immunomodulators, angiogenesis suppressors, substances suppressing tumor cells growth, such as DNA and RNA polynucleotides; chaperones HSP 60, HSP 70, HSP 90, tumor-specific peptides from the MAGE group, such as MAGE-A2, MAGE-A3, gp100, HER2/neu, EGFRvill or their fragments; cytostatic agents and other tumor growth inhibitors in the effective required doses

5. Protein-polypeptide complex for use in treating oncological skin diseases as a biologically active ingredient according to claim 1, **characterized in** systemic administration of the complex, apart from the local administration, subcutaneously once daily or once in 2-3 day during the courses of 10-14 days, 0.1-0.4 mg daily, with the 10-28-day intervals between the courses, depending on the severity of a neoplastic process, its localization, intensity of primary treatment and the local therapy course.

6. Protein-polypeptide complex for use in treating oncological skin diseases as a biologically active ingredient within the composition of the pharmaceutical (medical) preparation in the form of external applications after determination of the tumor size, directly on the tumor tissue and around its lesion prior to or after surgical removal, 3 -4 times per week within the course of 12-16 procedures; the protein-polypeptide complex is used in the concentration 0.05 - 0.4 mg together with the pharmaceutically acceptable carrier in the amount of 0.5 - 2.5 ml, along with that, the protein-polypeptide complex according to claim 1 is used.

7. Protein-polypeptide complex for use in treating oncological skin diseases as a biologically active ingredient within the composition of the pharmaceutical preparation, in the form of external application according to claim 6, **characterized in that**:
- For tumor size up to 1 cm, during one procedure 0.05-0.1 mg of the protein-polypeptide complex is used in 0.5-1.0 ml of the pharmaceutically acceptable carrier, 3-4 applications per week within the course of 12-16 procedures;
- For tumor size from 1 cm to 1.5 cm, during one procedure no more than 0.15 mg of the protein-polypeptide complex is used in 1.0-1.5 ml of the pharmaceutically acceptable carrier, 3-4 applications per week within the course of 12-16 procedures;
- For tumor size from 1.5 to 2.0 cm, during one procedure no more than 0.25-3.0 mg of the protein-polypeptide complex is used in 1.5-2.0 ml of the pharmaceutically acceptable diluent (carrier), 3-4 applications per week within the course of 12-16 procedures;
- For tumor size from 2.0 cm, during one procedure 0.4 mg of the protein-polypeptide complex is used in 2.0-3.0 ml of the pharmaceutically acceptable carrier, 4 applications per week within the course of 12-16 procedures.

8. Protein-polypeptide complex for use in treating oncological skin diseases as a biologically active ingredient within the composition of the pharmaceutical preparation used for external applications according to claim 6, **characterized in that** the complex is used in the form of ointment or gel, and a hydrophilic pharmaceutically acceptable carrier is used as ointment or gel base.

9. Protein-polypeptide complex for use in treating oncological skin diseases as a biologically active ingredient in the form of external applications according to claim 6 or claim 7, **characterized in that** the pharmaceutical preparation is used in the form of liposomal emulsion individually or in the form of ointment or gel with preservation of the same concentration and volumetric proportions, single and course dosage regimens.

10. Protein-polypeptide complex for use in treating oncological skin diseases as a biologically active ingredient within the composition of the pharmaceutical preparation for external applications according to claim 6 or claim 7, **characterized in that** the protein-polypeptide complex is used in combination with other immunomodulators, angiogenesis suppressors, substances suppressing tumor cells growth, such as DNA and RNA polynucleotides; chaperones HSP 60, HSP 70, HSP 90, tumor-specific peptides from the MAGE group, such as MAGE-A2, MAGE-A3, gp100, HER2/neu, EGFRvill or their fragments; cytostatic agents and other tumor growth inhibitors in the effective doses.

11. Protein-polypeptide complex for use in preventive treatment upon development of neoplastic processes of skin tissue, such as melanoma, basal-cell and squamous-cell cancer, including the use of pharmaceutical preparation containing protein-polypeptide complex and pharmaceutically acceptable carriers in the form of cosmetic compositions, such as day and night creams, gels, lotions individually or in the liposomal form, as well as in the form of photoactive or photo-protective compositions, daily, in the concentration 0.001-0.05 mg/ml, 1-2 times daily with the course dose of 10-20 days, at least 4 courses per year; along with that, the protein-polypeptide complex according to claim 1 is used.

## Patentansprüche

1. Protein-Polypeptid-Komplex zur Verwendung in der Behandlung onkologischer Hauterkrankungen als ein biologisch aktiver Inhaltsstoff innerhalb der Zusammensetzung der Arzneimittelanwendung in Form von 1,0-2,5 ml einer Lösung in einem pharmazeutisch akzeptablen Träger mit einer Komplexkonzentration darin in der Menge von 0,05 - 0,4 mg und injiziert mit dem Rücklauf der Nadel 1-3mal pro Woche in Form von 6-20 Injektionen im Verlauf von 4-14 Eingriffen während intrakutanen Injektionen um die Läsion des geschädigten Gewebes vor und/oder nach Tumorentfernung oder direkt in das Tumorgewebe nach Bestimmung der Tumorgröße; zusammen damit wird der Protein-Polypeptid-Komplex als Wirkstoff verwendet, der aus einem Homogenisat embryonaler Nerven- und Hautgewebe des Huftiers hergestellt wird und negativ geladene schwach saure und neutrale Proteine und Polypeptide mit der Molekülmasse von 5 bis 200 kDa enthält und einen Fraktionsgehalt mit der mittleren Molekülmasse innerhalb des Bereiches von 10 bis 120 kDa von mindestens 80%, allgemeinen Proteingehalt 0,2 - 4,2 mg/ml, maximalen Absorptionspeak des UV-Spektrums der Lösung bei der Wellenlänge 280 ± 5 nm, Peakpräsenz bei der Wellenlänge 274-284 nm im Bereich des UV-sichtbaren Spektrums, Vorhandensein spezifischer Banden im pl-Bereich von 4,2 bis 8,4 bei isoelektrischer Fokussierung in 5%-igem Polyacrylamidgel.

2. Protein-Polypeptid-Komplex zur Verwendung in der Behandlung onkologischer Hauterkrankungen als ein biologisch aktiver Inhaltsstoff innerhalb der Zusammensetzung der Arzneimittelanwendung nach Anspruch 1, **dadurch gekennzeichnet, dass** das Verdünnungsmittel enthaltend Pufferlösung und Hilfsstoffe als ein pharmazeutisch akzeptabler Träger verwendet wird.

3. Protein-Polypeptid-Komplex zur Verwendung in der Behandlung onkologischer Hauterkrankungen als ein biologisch aktiver Inhaltsstoff innerhalb der Zusammensetzung der Arzneimittelanwendung nach Anspruch 1, **dadurch gekennzeichnet, dass**:
- Bei einer Tumorgröße von bis zu 1 cm, ein Eingriff vor Entfernung der Läsion nicht mehr als 0,1 mg des Protein-Polypeptid-Komplexes in 1,0 ml des pharmazeutisch akzeptablen Verdünnungsmittels in Form von 6-8 Injektionen 1-2mal pro Woche verabreicht wird, der Verlauf beträgt 4-6 Eingriffe;
- Bei einer Tumorgröße von 1 cm bis 1,5 cm wird während eines Eingriffes nicht mehr als 0,15 mg des Protein-Polypeptid-Komplexes in 1,0-1,5 ml des pharmazeutisch akzeptablen Verdünnungsmittels in Form von 10-14 Injektionen 2-3mal pro Woche verabreicht;
- Bei einer Tumorgröße von 1,5 cm bis 2,0 cm wird während eines Eingriffes nicht mehr als 0,25 mg des Protein-Polypeptid-Komplexes in 1,5-2,0 ml des pharmazeutisch akzeptablen Verdünnungsmittels in Form von 12-16 Injektionen 2-3mal pro Woche verabreicht, der Verlauf beträgt 9-12 Eingriffe;
- Bei einer Tumorgröße ab 2,0 cm und größer wird während eines Eingriffes bis zu 0,4 mg des Protein-Polypeptid-Komplexes in 2,5-4,0 ml des pharmazeutisch akzeptablen Verdünnungsmittels in Form von 16-20 Injektionen mindestens dreimal pro Woche verabreicht, der Verlauf beträgt 12 Eingriffe.

4. Protein-Polypeptid-Komplex, verwendet zur Behandlung onkologischer Hauterkrankungen als ein biologisch aktiver Inhaltsstoff innerhalb der Zusammensetzung der Arzneimittelanwendung nach Anspruch 1, **dadurch gekennzeichnet, dass** er in Kombination mit anderen Immunmodulatoren, Angiogenesesuppressoren, Substanzen, die das Wachstum von Tumorzellen unterdrücken wie DNA- und RNA-Polynukleotide; Chaperonen HSP 60, HSP 70, HSP 90, tumorspezifischen Peptiden aus der MAGE-Gruppe, wie MAGE-A2, MAGE-A3, gp 100, HER2/neu, EGFRvill oder deren Fragmenten; zytostatischen Mitteln und anderen Inhibitoren des Tumorwachstums in den effektiv erforderlichen Dosen verabreicht wird.

5. Protein-Polypeptid-Komplex zur Verwendung in der Behandlung onkologischer Hauterkrankungen als ein biologisch aktiver Inhaltsstoff nach Anspruch 1, **gekennzeichnet durch** systemische Verabreichung des Komplexes, separat von der lokalen Verabreichung, subkutan einmal täglich oder einmal in 2-3 Tagen während der Verläufe von 10-14 Tagen, 0,1-0,4 mg täglich, mit den 10-28-tägigen Intervallen zwischen den Verläufen, in Abhängigkeit von der Schwere eines neoplastischen Prozesses, seiner Lokalisation, Intensität der Primärbehandlung und dem lokalen Therapieverlauf.

6. Protein-Polypeptid-Komplex zur Verwendung in der Behandlung onkologischer Hauterkrankungen als ein biologisch aktiver Inhaltsstoff innerhalb der Zusammensetzung der pharmazeutischen (medizinischen) Zubereitung in Form externer Applikationen nach Bestimmung der Tumorgröße, direkt auf das Tumorgewebe und um seine Läsion vor oder nach chirurgischer Entfernung, 3-4 mal pro Woche im Verlauf von 12-16 Eingriffen; der Protein-Polypeptid-Komplex wird in der Konzentration von 0,05 - 0,4 mg gemeinsam mit dem pharmazeutisch akzeptablen Träger in der Menge von 0,5 - 2,5 ml verwendet, zusammen damit wird der Protein-Polypeptid-Komplex nach Anspruch 1 verwendet.

7. Protein-Polypeptid-Komplex zur Verwendung in der Behandlung onkologischer Hauterkrankungen als ein biologisch aktiver Inhaltsstoff innerhalb der Zusammensetzung der pharmazeutischen Zubereitung in Form externer Applikation nach Anspruch 6, **dadurch gekennzeichnet, dass**:
- Bei einer Tumorgröße von bis zu 1 cm wird während eines Eingriffes 0,05-0,1 mg des Protein-Polypeptid-Komplexes in 0,5-1,0 ml des pharmazeutisch akzeptablen Trägers verwendet, 3-4 Applikationen pro Woche innerhalb des Verlaufes von 12-16 Eingriffen;
- Bei einer Tumorgröße von 1 cm bis 1,5 cm wird während eines Eingriffes nicht mehr als 0,15 mg des Protein-Polypeptid-Komplexes in 1,0-1,5 ml des pharmazeutisch akzeptablen Trägers verwendet, 3-4 Anwendungen pro Woche innerhalb des Verlaufes von 12-16 Eingriffen;
- Bei einer Tumorgröße von 1,5 bis 2,0 cm wird während eines Eingriffes nicht mehr als 0,25-3,0 mg des Protein-Polypeptid-Komplexes in 1,5-2,0 ml des pharmazeutisch akzeptablen Verdünnungsmittels (Trägers) verwendet, 3-4 Applikationen pro Woche innerhalb des Verlaufes von 12-16 Eingriffen;
- Bei einer Tumorgröße ab 2,0 cm wird während eines Eingriffes 0,4 mg des Protein-Polypeptid-Komplexes in 2,0-3,0 ml des pharmazeutisch akzeptablen Trägers verwendet, 4 Applikationen pro Woche innerhalb des Verlaufes von 12-16 Eingriffen.

8. Protein-Polypeptid-Komplex zur Verwendung in der Behandlung onkologischer Hauterkrankungen als ein biologisch aktiver Inhaltsstoff innerhalb der Zusammensetzung der pharmazeutischen Zubereitung, verwendet für externe Applikationen nach Anspruch 6, **dadurch gekennzeichnet, dass** der Komplex in Form einer Salbe oder eines Gels verwendet wird und ein hydrophiler pharmazeutisch akzeptabler Träger als Salben- oder Gelgrundlage verwendet wird.

9. Protein-Polypeptid-Komplex zur Verwendung in der Behandlung onkologischer Hauterkrankungen als ein biologisch aktiver Inhaltsstoff in Form externer Applikationen nach Anspruch 6 oder Anspruch 7, **dadurch gekennzeichnet, dass** die pharmazeutische Zubereitung in Form einer liposomalen Emulsion individuell oder in Form einer Salbe oder eines Gels unter Beibehaltung der gleichen Konzentration und der gleichen Volumenverhältnisse, Einzel- und Verlaufsdosierungsregimes verwendet wird.

10. Protein-Polypeptid-Komplex zur Verwendung in der Behandlung onkologischer Hauterkrankungen als ein biologisch aktiver Inhaltsstoff innerhalb der Zusammensetzung der pharmazeutischen Zubereitung für externe Applikationen nach Anspruch 6 oder 7, **dadurch gekennzeichnet, dass** der Protein-Polypeptid-Komplex in Kombination mit anderen Immunmodulatoren, Angiogenesesuppressoren, Substanzen, die das Wachstum von Tumorzellen unterdrücken wie DNA- und RNA-Polynukleotide; Chaperonen HSP 60, HSP 70, HSP 90, tumorspezifischen Peptiden aus der MAGE-Gruppe, wie MAGE-A2, MAGE-A3, gp 100, HER2/neu, EGFRvill oder deren Fragmenten; zytostatischen Mitteln und anderen Inhibitoren des Tumorwachstums in den effektiven Dosen verwendet wird.

11. Protein-Polypeptid-Komplex zur Verwendung in der vorbeugenden Behandlung nach der Entwicklung neoplastischer Prozesse von Hautgewebe, wie Melanom, Basalzell- und Plattenepithelkarzinom, beinhaltend die Verwendung einer pharmazeutischen Zubereitung, die den Protein-Polypeptid-Komplex und pharmazeutisch akzeptable Träger enthält, in Form kosmetischer Zusammensetzungen, wie zum Beispiel als Tages- und Nachtcremes, Gele, Lotionen, einzeln oder in der liposomalen Form, sowie in Form photoaktiver oder photoschützender Zusammensetzungen, täglich, in der Konzentration 0,001-0,05 mg/ml, 1-2 mal täglich bei der Verlaufsdosis von 10-20 Tagen, mindestens 4 Verläufe pro Jahr; zusammen damit wird der Protein-Polypeptid-Komplex gemäß Anspruch 1 verwendet.

## Revendications

1. Complexe protéine-polypeptide pour utilisation dans le traitement de maladies cutanées oncologiques en tant qu'ingrédient biologiquement actif dans la composition du médicament sous la forme de 1,0 à 2,5 ml de solution dans un véhicule pharmaceutiquement acceptable avec une concentration de complexe dans celle-ci de 0,05 à 0,4 mg, injectée par course inversée de l'aiguille 1 à 3 fois par semaine sous la forme de 6 à 20 injections pendant une cure de 4 à 14 procédures d'injections intracutanées autour d'une lésion du tissu endommagé avant et/ou après retrait d'une tumeur, ou directement dans le tissu tumoral après détermination de la taille de la tumeur ; conjointement avec cela, le complexe protéine-polypeptide est utilisé en tant que principe actif, est produit à partir d'homogénat de tissus cutanés et nerveux embryonnaires d'ongulés d'élevage et contient des polypeptides et des protéines neutres et légèrement acides chargés négativement ayant une masse moléculaire de 5 à 200 kDa avec une teneur en la fraction ayant une masse moléculaire moyenne située dans la plage allant de 10 à 120 kDa d'au moins 80 %, une teneur en protéine totale de 0,2 à 4,2 mg/ml, un pic d'absorption maximal du spectre UV en solution à la longueur d'onde de 280 ± 5 nm, une présence de pic à la longueur d'onde de 274 à 284 nm dans la gamme du spectre UV-visible, la présence de bandes spécifiques dans la plage p1 de 4,2 à 8,4 pour une focalisation isoélectrique dans du gel à 5 % de polyacrylamide.

2. Complexe protéine-polypeptide pour utilisation dans le traitement de maladies cutanées oncologiques en tant qu'ingrédient biologiquement actif dans la composition du médicament selon la revendication 1, **caractérisé en ce que** le diluant contenant une solution tampon et des excipients est utilisé en tant que véhicule pharmaceutiquement acceptable.

3. Complexe protéine-polypeptide pour utilisation dans le traitement de maladies cutanées oncologiques en tant qu'ingrédient biologiquement actif dans la composition du médicament selon la revendication 1, **caractérisé en ce que** :
- pour une taille de tumeur allant jusqu'à 1 cm, durant une procédure avant ablation d'une lésion, au plus 0,1 mg du complexe protéine-polypeptide dans 1,0 ml du diluant pharmaceutiquement acceptable est administré sous la forme de 6 à 8 injections 1 ou 2 fois par semaine, et la cure est de 4 à 6 procédures ;
- pour une taille de tumeur de 1 à 1,5 cm, durant 1 procédure, au plus 0,15 mg du complexe protéine-polypeptide dans 1,0 à 1,5 ml du diluant pharmaceutiquement acceptable est administré sous la forme de 10 à 14 injections 2 à 3 fois par semaine ;
- pour une taille de tumeur de 1,5 à 2,0 cm, durant une procédure, au plus 0,25 mg du complexe protéine-polypeptide dans 1,5 à 2,0 ml du diluant pharmaceutiquement acceptable est administré sous la forme de 12 à 16 injections 2 à 3 fois par semaine, et la cure est de 9 à 12 procédures ;
- pour une taille de tumeur de 2,0 cm ou plus, durant une procédure, jusqu'à 0,4 mg du complexe protéine-polypeptide dans 2,5 à 4,0 ml du diluant pharmaceutiquement acceptable est administré sous la forme de 16 à 20 injections au moins 3 fois par semaine, et la cure est de 12 procédures.

4. Complexe protéine-polypeptide utilisé dans le traitement de maladies cutanées oncologiques en tant qu'ingrédient biologiquement actif dans la composition du médicament selon la revendication 1, **caractérisé en ce qu'**il est administré en combinaison avec d'autres immunomodulateurs, des suppresseurs d'angiogenèse, des substances supprimant la croissance de cellules tumorales, telles que des polynucléotides d'ADN et d'ARN ; des chaperons HSP 60, HSP 70, HSP 90, des peptides spécifiques d'une tumeur du groupe MAGE, tels que MAGE-A1, MAGE-A2, MAGE-A3, gp100, HER2/neu, EGFRvill ou leurs fragments ; des agents cytostatiques et d'autres inhibiteurs de croissance tumorale en les doses efficaces requises.

5. Complexe protéine-polypeptide pour utilisation dans le traitement de maladies cutanées oncologiques en tant qu'ingrédient biologiquement actif selon la revendication 1, **caractérisé par** l'administration systémique du complexe, à part l'administration locale sous-cutanée, une fois par jour ou une fois tous les 2 ou 3 jours durant des cures de 10 à 14 jours, à raison de 0,1 à 0,4 mg par jour, avec des intervalles de 10 à 28 jours entre les cures, en fonction de la gravité d'un processus néoplasique, de sa localisation, de l'intensité d'un traitement primaire et de la cure thérapeutique locale.

6. Complexe protéine-polypeptide pour utilisation dans le traitement de maladies cutanées oncologiques en tant qu'ingrédient biologiquement actif dans la composition de la préparation pharmaceutique (médicale) sous la forme d'applications externes après détermination de la taille d'une tumeur, directement sur le tissu tumoral et autour de sa lésion avant ou après ablation chirurgicale, 3 à 4 fois par semaine lors d'une cure de 12 à 16 procédures ; le complexe protéine-polypeptide est utilisé à une concentration de 0,05 à 0,4 mg conjointement avec le véhicule pharmaceutiquement acceptable en une quantité de 0,5 à 2,5 ml et, conjointement avec cela, le complexe protéine-polypeptide selon la revendication 1 est utilisé.

7. Complexe protéine-polypeptide pour utilisation dans le traitement de maladies cutanées oncologiques en tant qu'ingrédient biologiquement actif dans la composition de la préparation pharmaceutique, sous la forme d'une application externe selon la revendication 6, **caractérisé en ce que** :
- pour une taille de tumeur allant jusqu'à 1 cm, durant une procédure, 0,05 à 0,1 mg du complexe protéine-polypeptide est utilisé dans 0,5 à 1,0 ml du véhicule pharmaceutiquement acceptable, à raison de 3 à 4 applications par semaine lors d'une cure de 12 à 16 procédures ;
- pour une taille de tumeur de 1 cm à 1,5 cm, durant une procédure, au plus 0,15 mg du complexe protéine-polypeptide est utilisé dans 1,0 à 1,5 ml du véhicule pharmaceutiquement acceptable, à raison de 3 à 4 applications par semaine lors d'une cure de 12 à 16 procédures ;
- pour une taille de tumeur de 1,5 à 2,0 cm, durant une procédure, au plus 0,25 à 3,0 mg du complexe protéine-polypeptide sont utilisés dans 1,5 à 2,0 ml du diluant pharmaceutiquement acceptable (véhicule), à raison de 3 à 4 applications par semaine lors d'une cure de 12 à 16 procédures ;
- pour une taille de tumeur de 2,0 cm ou plus, durant une procédure, 0,4 mg du complexe protéine-polypeptide est utilisé dans 2,0 à 3,0 ml du diluant pharmaceutiquement acceptable, à raison de 4 applications par semaine lors d'une cure de 12 à 16 procédures.

8. Complexe protéine-polypeptide pour utilisation dans le traitement de maladies cutanées oncologiques en tant qu'ingrédient biologiquement actif dans la composition de la préparation pharmaceutique utilisée pour des applications externes selon la revendication 6, **caractérisé en ce que** le complexe est utilisé sous la forme d'une pommade ou d'un gel, et un véhicule pharmaceutiquement acceptable hydrophile est utilisé en tant que base de pommade ou de gel.

9. Complexe protéine-polypeptide pour utilisation dans le traitement de maladies cutanées oncologiques en tant qu'ingrédient biologiquement actif sous la forme d'applications externes selon la revendication 6 ou la revendication 7, **caractérisé en ce que** la préparation pharmaceutique est utilisée sous la forme d'une émulsion de liposomes individuellement ou sous la forme d'une pommade ou d'un gel avec conservation de la même concentration et des mêmes proportions volumétriques, en régimes posologiques uniques et en cure.

10. Complexe protéine-polypeptide pour utilisation dans le traitement de maladies cutanées oncologiques en tant qu'ingrédient biologiquement actif dans la composition de la préparation pharmaceutique pour applications externes selon la revendication 6 ou la revendication 7, **caractérisé en ce que** ce le complexe de protéine-polypeptide est utilisé en combinaison avec d'autres immunomodulateurs, des suppresseurs d'angiogenèse, des substances supprimant la croissance de cellules tumorales, telles que des polynucléotides d'ADN et d'ARN ; des chaperons HSP 60, HSP 70, HSP 90, des peptides spécifiques d'une tumeur du groupe MAGE, tels que MAGE-Al, MAGE-A2, MAGE-A3, gp100, HER2/neu, EGFRvill ou leurs fragments ; des agents cytostatiques et d'autres inhibiteurs de croissance tumorale en les doses efficaces requises.

11. Complexe protéine-polypeptide pour utilisation dans un traitement préventif suite au développement de processus néoplasiques du tissu cutané, tels qu'un mélanome, un cancer à cellules basales et à cellules squameuses, y compris l'utilisation d'une préparation pharmaceutique contenant un complexe protéine-polypeptide et des véhicules pharmaceutiquement acceptables sous la forme de compositions cosmétiques, telles que des crèmes de jour et de nuit, des gels, des lotions, individuellement ou sous forme de liposomes, ainsi que sous la forme de compositions photoactives ou photoprotectrices, chaque jour, à une concentration de 0,001 à 0,05 mg/ml, 1 ou 2 fois par jour sur une cure de 10 à 20 jours, avec au moins 4 cures par an ; et conjointement avec cela, le complexe protéine-polypeptide selon la revendication 1 est utilisé.
